# EUROPEAN PATENT APPLICATION

(11) **EP 4 501 900 A1**
(43) Date of publication of application: **05.02.2025**
(21) Application number: 23780112.1
(22) Date of filing: 24.03.2023
(51) Int. Cl.: C07C 67/29, C08G 63/181, C08G 63/78

(54) **METHOD FOR PRODUCING AROMATIC BIS(HYDROXYALKYL) DICARBOXYLATE AND METHOD FOR PRODUCING RECYCLED AROMATIC POLYESTER**

(30) Priority: 29.03.2022 JP 2022053234
(71) Applicant: TEIJIN FRONTIER CO., LTD., Osaka-shi, Osaka 530-0005 (JP)
(72) Inventor: TSURUDA Ryo, Osaka-shi, Osaka 530-0005 (JP); SUNOUCHI Satoshi, Osaka-shi Osaka 530-0005 (JP)
(74) Representative: Carpmaels & Ransford LLP
(86) International application number: PCT/JP2023/011694
(87) International publication number: WO 2023/190102

(57) **Abstract**

Provided are a method for producing an aromatic bis(hydroxyalkyl) dicarboxylate, comprising subjecting a polyester to depolymerization in the presence of a catalyst, in which the catalyst is a manganese catalyst, and in which the amount of the catalyst used is 20 to 500 mmol%, based on the mole of the polyester, a method for producing an aromatic bis(hydroxyalkyl) dicarboxylate by subjecting a polyester to depolymerization by the method, and a method for producing a polyester polymer by subjecting the aromatic bis(hydroxyalkyl) dicarboxylate as a raw material to repolymerization, in which these methods are a method for producing a polyester polymer having as small the degree of discoloration as that of a virgin polymer (which is produced by polymerizing a monomer, and which has not experienced depolymerization) of polyester and a method for producing an intermediate of the polyester polymer.

## Description

### Technical Field

The present invention relates to a method for producing an aromatic bis(hydroxyalkyl) dicarboxylate by subjecting a polyester to depolymerization and a method for producing an aromatic polyester using the aromatic bis(hydroxyalkyl) dicarboxylate, more particularly relates to a method for producing a recycled aromatic polyester, comprising subjecting to depolymerization an aromatic polyester contained in waste polyester, a recovered product, or the like, which contains an aromatic polyester, and subjecting the resultant component to repolymerization, obtaining a recycled aromatic polyester.

### Background Art

Aromatic polyester, for example, polyethylene terephthalate has excellent properties, and has been widely used, for example, as a fiber, a film, and a resin molded article. In the production process and processing step for the polyester, a residual virgin polyester raw material, waste polyester in a fiber form, a film form, or another form, and discarded materials remain as waste. Further, the fibers, films, and resin molded articles produced as polyester products are disposed of after used or in the residual virgin state, and such waste materials cause a problem of adversely affecting the environment.

As a method for reusing the waste or discarded materials caused in the process, residual virgin raw materials, and waste products, there are material recycling, chemical recycling, and thermal recycling methods. Among the chemical recycling, there is a chemical recycling method using a monomer in a form such that one molecule of aromatic dicarboxylic acid and two molecules of diol are bonded through ester bonds, or using repolymerization of an intermediate obtained by depolymerization to an oligomer having the monomer ester bonded, and this method is such excellent that a recycled aromatic polyester can be directly produced by a polycondensation reaction and thus less energy is required.

As an example of the above-mentioned method, there is a method in which polyethylene terephthalate is subjected to depolymerization in ethylene glycol to obtain bis(2-hydroxyethyl) terephthalate, and the bis(2-hydroxyethyl) terephthalate is subjected to polycondensation, obtaining a recycled aromatic polyester.

However, there is a problem in that the thus obtained recycled aromatic polyester polymer has suffered discoloration. Further, another problem is caused as follows. It is likely that the aromatic polyester contains, for example, for the purpose of improving the dyeability of a polyester fiber, a metal sulfoisophthalate, such as sodium sulfoisophthalate, or a sulfoisophthalic acid component, such as a tetraalkylphosphonium sulfoisophthalate or tetraalkylammonium sulfoisophthalate, as a dicarboxylic acid component, and, in such a case, the recycled aromatic polyester obtained by merely repolymerization after depolymerization has, in addition to the problem of discoloration, a further problem in that the molecular weight (intrinsic viscosity) or melting point is reduced and the crystalline properties become poor, or an increased amount of by-products (such as diethylene glycol) are generated, leading to poor heat resistance.

Under the circumstances, in PTL 1, with respect to the step for removing a discoloration causative agent, attempts are made, for example, an adsorption treatment in which the discoloration causative agent is permitted to be in contact with an adsorbent, a decomposition treatment in which the discoloration causative agent is decomposed using a decomposer, and a reduction treatment in which the discoloration causative agent is reduced using a reducing agent. However, removal of a discoloration causative agent, such as a dye, clearly mixed into the polymer is achieved to some extent, but a method for obtaining a polyester polymer which has as small a degree of discoloration as a polyester polymer generally produced has not yet been obtained.

PTL 1: JP2008-88096A

### Summary of Invention

### Technical Problem

In view of the above, the present invention has been made. The first object of the present invention is to provide a method for producing an aromatic bis(hydroxyalkyl) dicarboxylate, which comprises subjecting a polyester to depolymerization, and a method for producing a polyester polymer from the obtained aromatic bis(hydroxyalkyl) dicarboxylate, in which these methods are a method for producing an aromatic polyester polymer having as small the degree of discoloration as that of a virgin polymer (which is produced by polymerizing a monomer, and which has not experienced depolymerization) of aromatic polyester and a method for producing an intermediate of the aromatic polyester polymer.

The second object of the present invention is to provide a method for producing a recycled aromatic polyester, which includes subjecting to depolymerization an aromatic polyester contained in a waste polyester fiber, a recovered polyester product, or the like, which contains an aromatic polyester, and subjecting the resultant component to repolymerization, obtaining a recycled aromatic polyester, in which the method is advantageous in that the recycled aromatic polyester produced by the method has as excellent a heat resistance as that of a virgin polymer of aromatic polyester. Particularly, the object of the present invention is to provide a method for producing a recycled aromatic polyester, which is advantageous in that even when a sulfoisophthalic acid component is contained as the dicarboxylic acid component in the aromatic polyester, the produced recycled aromatic polyester has as excellent a heat resistance as that of a virgin polymer of aromatic polyester. Solution to Problem

Specifically, the first invention of the present invention is a method for producing an aromatic bis(hydroxyalkyl) dicarboxylate, including subjecting a polyester to depolymerization in the presence of a catalyst, in which the catalyst is a manganese catalyst, and in which the amount of the catalyst used is 20 to 500 mmol%, based on the mole of the polyester.

The method for producing an aromatic bis(hydroxyalkyl) dicarboxylate of the present invention is a method for producing an aromatic bis(hydroxyalkyl) dicarboxylate, including subjecting a polyester to depolymerization in the presence of a catalyst, in which the catalyst is a manganese catalyst, and in which the amount of the catalyst used is 20 to 500 mmol%, based on the mole of the polyester.

It is preferred that the polyester is included mainly of a polyalkylene terephthalate, and the polyalkylene terephthalate is preferably one of polyethylene terephthalate, polytrimethylene terephthalate, and polybutylene terephthalate.

Further, it is preferred that the aromatic bis(hydroxyalkyl) dicarboxylate is a bis(hydroxyalkyl) benzenedicarboxylate, that the manganese catalyst is manganese acetate, that the catalyst is used in the form of a solution obtained by preliminarily dissolving the catalyst in an alkylene glycol, and that, after the depolymerization, the aromatic bis(hydroxyalkyl) dicarboxylate is subjected to crystal deposition in an alkylene glycol by decreasing the temperature, and, after the crystal deposition, the resultant aromatic bis(hydroxyalkyl) dicarboxylate is preferably washed with water.

The first invention of the present invention also includes a method for producing a polyester resin, which includes subjecting to repolymerization an aromatic bis(hydroxyalkyl) dicarboxylate obtained by any of the above-mentioned methods. The first invention also includes a polyester resin obtained by the above method.

A preferred embodiment of the invention is a method for producing a recycled aromatic polyester, including subjecting a polyester containing an aromatic polyester to depolymerization, and then subjecting the component obtained by depolymerization to polycondensation, obtaining a recycled aromatic polyester, in which the depolymerization is performed by conducting depolymerization of the polyester in the presence of a catalyst to obtain an aromatic bis(hydroxyalkyl) dicarboxylate, in which the catalyst used in the depolymerization is a manganese catalyst, and in which the amount of the catalyst used is 20 to 500 mmol%, based on the mole of the polyester.

In the preferred embodiment, it is further preferred that, after the depolymerization and before the polycondensation, the aromatic bis(hydroxyalkyl) dicarboxylate is purified.

It is especially preferred that the polycondensation is performed by conducting polycondensation of the purified aromatic bis(hydroxyalkyl) dicarboxylate in the presence of an alkali metal and/or an alkaline earth metal.

The present invention is also directed to a method for producing a recycled aromatic polyester, including subjecting a polyester containing an aromatic polyester to depolymerization, and then subjecting the component obtained by depolymerization to polycondensation, obtaining a recycled aromatic polyester, in which the polyester containing an aromatic polyester is subjected to depolymerization in an alkylene glycol to obtain an aromatic bis(hydroxyalkyl) dicarboxylate, and the aromatic bis(hydroxyalkyl) dicarboxylate is purified, and the purified aromatic bis(hydroxyalkyl) dicarboxylate is subjected to polycondensation in the presence of an alkali metal and/or an alkaline earth metal, obtaining a recycled aromatic polyester. This is the second invention of the present invention and described in detail later. Advantageous Effects of Invention

In the present invention, firstly, there can be provided a method for producing an aromatic bis(hydroxyalkyl) dicarboxylate, which includes subjecting a polyester to depolymerization, and a method for producing a polyester polymer from the obtained aromatic bis(hydroxyalkyl) dicarboxylate, in which these methods are a method for producing a polyester polymer having as small the degree of discoloration as that of a virgin polymer (which is produced by polymerizing a monomer, and which has not experienced depolymerization) of polyester and a method for producing an intermediate of the polyester polymer.

In the present invention, secondly, there can be provided a method for producing a recycled aromatic polyester, including subjecting to depolymerization an aromatic polyester contained in a waste polyester fiber, a recovered polyester product, or the like, which contains an aromatic polyester, and subjecting the resultant component to repolymerization, obtaining a recycled aromatic polyester, in which the method is advantageous in that the recycled aromatic polyester produced by the method has as excellent a heat resistance as that of a virgin polymer of aromatic polyester. Particularly, there can be provided a method for producing a recycled aromatic polyester, which is advantageous in that even when a sulfoisophthalic acid component is contained as the dicarboxylic acid component in the aromatic polyester, the produced recycled aromatic polyester has as excellent a heat resistance as that of a virgin polymer of aromatic polyester.

### Description of Embodiments

### [First invention]

Hereinbelow, the first invention of the present invention will be described in detail.

The invention is a method for producing an aromatic bis(hydroxyalkyl) dicarboxylate, which includes subjecting a polyester to depolymerization in the presence of a catalyst, and the polyester means a polycondensation product synthesized by subjecting a polycarboxylic acid and a polyalcohol to dehydration condensation to form ester bonds. Further, the polyester is a polymer having ester bonds. The polyester is preferably a semi-aromatic polyester.

With respect to the polycarboxylic acid constituting the polyester, a dicarboxylic acid or an ester-forming derivative thereof is preferably used, and specific examples include aromatic dicarboxylic acids, such as terephthalic acid, isophthalic acid, phthalic acid, 2,6-naphthalenedicarboxylic acid, 1,5-naphthalenedicarboxylic acid, bis(p-carboxyphenyl)methane, anthracenedicarboxylic acid, 4,4'-diphenyl ether dicarboxylic acid, tetrabutylphosphonium 5-isophthalate, and sodium 5-sulfoisophthalate. Further examples include aliphatic dicarboxylic acids, such as oxalic acid, succinic acid, adipic acid, sebacic acid, azelaic acid, dodecanedioic acid, malonic acid, glutaric acid, and dimer acid, and alicyclic dicarboxylic acids, such as 1,3-cyclohexanedicarboxylic acid and 1,4-cyclohexanedicarboxylic acid. Further, an ester-forming derivative of the dicarboxylic acid can be used.

Especially, with respect to the polycarboxylic acid constituting the polyester used in the invention, terephthalic acid or 2,6-naphthalenedicarboxylic acid is more preferably mainly used. Further, terephthalic acid or 2,6-naphthalenedicarboxylic acid is preferably applied to the polyester using terephthalic acid as a main component and an isophthalic acid component as a copolymerizable component, more specifically to the polyester having copolymerized therewith isophthalic acid or sodium 5-sulfoisophthalate.

With respect to the polyalcohol which is another one component constituting the polyester, a diol or an ester-forming derivative thereof is preferably used, and specific examples include aliphatic glycols having 2 to 20 carbon atoms, such as ethylene glycol, 1,3-propanediol, propylene glycol, 1,4-butanediol, neopentyl glycol, 1,5-pentanediol, 1,6-hexanediol, decamethylene glycol, cyclohexanedimethanol, cyclohexanediol, and dimer diol.

Alternatively, as the polyalcohol, a long-chain glycol having a molecular weight of 200 to 100,000, specifically, polyethylene glycol, poly-1,3-propylene glycol, poly-1,2-propylene glycol, polytetramethylene glycol, or the like can be used. Further, an aromatic dioxy compound, specifically, 4,4'-dihydroxybiphenyl, hydroquinone, tert-butylhydroquinone, bisphenol A, bisphenol S, bisphenol F, or the like can be used. An ester-forming derivative of the polyalcohol or diol is preferably used.

Especially, with respect to the alcohol constituting the polyester used in the invention, ethylene glycol (hereinafter, frequently abbreviated to "EG"), 1,3-propanediol, or 1,4-butanediol is preferably used.

In the invention, the polyester using a combination of the above-mentioned polycarboxylic acid and polyalcohol can be used as a starting material, and the polyester is preferably a semi-aromatic polyester, further preferably a polyalkylene terephthalate. It is especially preferred that the polyalkylene terephthalate is one of polyethylene terephthalate, polytrimethylene terephthalate, and polybutylene terephthalate.

The method for producing an aromatic bis(hydroxyalkyl) dicarboxylate of the invention includes subjecting the above-mentioned polyester to depolymerization in the presence of a catalyst, and, in the invention, it is important to select a manganese catalyst as the catalyst.

Examples of manganese catalysts include a fatty acid salt, a carbonate, a sulfate, a phosphate, an oxide, a hydroxide, a halide, or an alcoholate of manganese (Mn), and these catalysts are used individually or preferably in combination. In the invention, among these, manganese oxide or manganese acetate can be used, and manganese acetate is especially preferably used.

Further, the catalyst used is preferably used in the form of a solution obtained by preliminarily dissolving the catalyst in an alkylene glycol. With respect to the alkylene glycol (hereinafter, frequently abbreviated to "AG"), the same alkylene glycol as the diol component forming the main chain of the above-mentioned polyester which is the starting raw material can be used. Further, there can be used the diol constituting the polyester which is finally obtained as a product by subjecting to repolymerization the aromatic bis(hydroxyalkyl) dicarboxylate obtained by the method of the invention.

With respect to the alkylene glycol forming or capable of forming the main chain of the polyester, for example, in the case where the polyester is polyethylene terephthalate (PET), examples of the alkylene glycols include ethylene glycol (EG), in the case where the polyester is polytrimethylene terephthalate, examples of the alkylene glycols include 1,3-propanediol (trimethylene glycol, C3G), and, in the case where the polyester is polybutylene terephthalate, examples of the alkylene glycols include 1,4-butanediol (C4G). Further, according to the purpose, a mixture of the above-mentioned alkylene glycols is preferably used as the alkylene glycol.

The reason is not clear, but only when a manganese catalyst is selected from various types of catalysts, the remarkable effects of the invention can be achieved. The effects of the invention are particularly actualized in depolymerization of a reused polyester containing no other coloring material rather than in depolymerization of a reused polyester containing other coloring material. For example, when a depolymerization product of polyester is stored for a long time, it is likely that the degree of discoloration of the product is gradually increased, but it is clear that the polyester product obtained by the method of the invention unlikely suffers discoloration.

Further, in the method of the invention, it is necessary that the amount of the catalyst used be 20 to 500 mmol%, based on the mole of the polyester. Further, the amount of the catalyst used is preferably 30 to 300 mmol%, especially preferably 50 to 150 mmol%. The unit "mol%" indicates the ratio of the number of the catalyst molecules to the number of the constituent units of the polyester, and "mmol%" means 1,000 times the value indicated by "mol%". When using the other general catalyst in such a small amount, satisfactory depolymerization cannot proceed, but the use of the manganese catalyst enables reduction of the amount of the catalyst used. When the amount of the catalyst used is smaller than the above range, the catalytic activity is not satisfactory, and, when the amount of the catalyst used is larger than the above range, the effect for suppressing discoloration is reduced.

In the method for producing an aromatic bis(hydroxyalkyl) dicarboxylate of the invention, which includes subjecting the above-mentioned polyester to depolymerization in the presence of a manganese catalyst, it is necessary that the amount of the catalyst used be 20 to 500 mmol%, based on the mole of the polyester.

In the method of the invention, it is preferred that, after conducting the depolymerization using the above-mentioned catalyst, the obtained aromatic bis(hydroxyalkyl) dicarboxylate is subjected to crystal deposition in an alkylene glycol by decreasing the temperature. With respect to the conditions for temperature decrease in the crystal deposition, preferred is a way of decreasing the temperature from a temperature of 60°C or higher to 25°C or lower, further preferably to 15°C or lower. It is preferred that, after the crystal deposition, the resultant aromatic bis(hydroxyalkyl) dicarboxylate is subjected to solid-liquid separation, and that the alkylene glycol content of the cake obtained after the solid-liquid separation is 100% by mass or less. The alkylene glycol content of the cake is further preferably 55% by mass or less, and controlled to be in the range of from 1 to 30% by mass, especially preferably in the range of from 5 to 25% by mass. Further, the amount of the alkylene glycol used in the first depolymerization is preferably 2 to 20 times, further preferably 3 to 10 times the amount of the polyester which is a raw material. In the method of the invention, by using an increased amount of the alkylene glycol in the depolymerization and conducting crystal deposition and solid-liquid separation, the amount of the depolymerization catalyst and other foreign matter mixed into the product can be further reduced.

The amount of the manganese catalyst used in the invention is small, and therefore the above-mentioned treatment in an alkylene glycol can further reduce the amount of the catalyst. Further, especially when manganese acetate is used as the catalyst, the solubility of manganese acetate in an alkylene glycol is such high that the amount of the remaining catalyst in the subsequent step can be more effectively reduced.

Further, in the method of the invention, it is preferred that the cake obtained after the depolymerization is subjected to the above-mentioned crystal deposition and then washed with water or an alkylene glycol. Further, it is preferred that a washing treatment is conducted while spraying a washing liquid onto the cake using a Nutsche (Buchner funnel). By performing these treatments, the depolymerization catalyst, other discoloration causative agents and the like dissolved in the alkylene glycol are washed away, making it possible to obtain the aromatic bis(hydroxyalkyl) dicarboxylate having a higher degree of purification. With respect to the solution used for washing, a solution having a low viscosity is preferred, and, from this point of view, water is preferably used. The amount of the washing liquid is preferably 1 to 100 times, further preferably 1.5 to 10 times the weight of the cake. The temperature of the washing liquid is preferably in the range of from 0 to 40°C. When the temperature is too high, the cake per se is likely to be dissolved, so that the yield is lowered. Then, the cake is dried by means of a vacuum dryer or the like, so that the aromatic bis(hydroxyalkyl) dicarboxylate can be obtained. With respect to the alkylene glycol used in the method of the invention, when the alkylene glycol is the same as the diol component of the polyester resin obtained after the repolymerization, a preferred method for producing a polyester is such that the cake is not dried but is as such subjected to repolymerization.

The aromatic bis(hydroxyalkyl) dicarboxylate obtained by the method of the invention varies depending on the type of the polyester or alkylene glycol used, but, when a polyester (polyalkylene terephthalate) using mainly terephthalic acid as the polycarboxylic acid is used as a raw material, preferred is the method for producing a bis(hydroxyalkyl) benzenedicarboxylate (hereinafter, frequently referred to as "BHAT; bishydroxyalkyl terephthalate"). More specifically, when C3G (1,3-propanediol (trimethylene glycol)) is used as the alkylene glycol, BHPT (bishydroxypropyl terephthalate) is produced, and, when C4G (1,4-butanediol) is used as the alkylene glycol, BHBT (bishydroxybutyl terephthalate) is produced. Particularly, when using, as a component constituting the polyester, polyethylene terephthalate mainly formed from terephthalic acid and ethylene glycol, bis(hydroxyethyl) benzenedicarboxylate (BHET; bishydroxyethyl terephthalate) can be produced.

Further, by subjecting the aromatic bis(hydroxyalkyl) dicarboxylate obtained by the method of the invention to repolymerization according to a conventionally known method, there can be obtained a polyester resin which is unlikely to suffer discoloration and which has excellent hue.

That is, the method for producing a polyester resin, which is another invention of the present invention, is a method which includes subjecting the aromatic bis(hydroxyalkyl) dicarboxylate obtained by the above-described method to repolymerization to produce a polyester resin. The polyester resin, which is still another invention of the present invention, is obtained by the above method for producing a polyester resin.

With respect to the catalyst used in repolymerization conducted for obtaining a polyester resin, a known Sb, Ge, or titanium catalyst or the like can be used, and particularly, diantimony trioxide is preferably used. It is preferred that a polycondensation reaction is conducted while distilling off an alkylene glycol and the like generated by a reaction during the repolymerization out of the reaction vessel. The amount of the catalyst used is preferably in the range of from 10 to 1,000 ppm, based on the weight of the aromatic bis(hydroxyalkyl) dicarboxylate used.

Further, in the method for producing a polyester resin of the invention, after the polycondensation using a catalyst, a conventionally known phosphorus stabilizer is preferably used. The amount of the phosphorus stabilizer used is preferably in the range of from 1 to 100 ppm, based on the weight of the aromatic bis(hydroxyalkyl) dicarboxylate used.

The thus obtained polyester resin is a resin which is unlikely to suffer discoloration, such as yellowing, differing from a polyester resin obtained using Mg hydroxide, Na hydroxide, or the like, which has conventionally been generally used, as a depolymerization catalyst. Discoloration of the aromatic bis(hydroxyalkyl) dicarboxylate obtained in the intermediate stage is as small as it can be visually recognized, but hue L*, a*, and b* values of the resin obtained after repolymerization are measured using a colorimeter, and particularly discoloration is remarkable from the b* value, and, whereas the polyester produced under the conditions in the invention has a b* value as low as -3 or less, further preferably - 3.5 or less, the polyester produced using other catalysts has a b* value higher than the above value, or has a positive value (strong yellowness). The reason for this is not clarified, but it is considered that the manganese catalyst has effective advantages in that the manganese catalyst can cause depolymerization in a low concentration, in that the manganese catalyst is unlikely to form a coloring by-product, and in that dissociation of the manganese catalyst from the aromatic bis(hydroxyalkyl) dicarboxylate is easy in the subsequent crystal deposition step and the like, so that the catalyst is very unlikely to remain as an impurity.

Further, the obtained polyester resin can be advantageously used in applications, such as a fiber, a film, and a resin.

### [Second invention]

The second invention of the present invention is described below in detail. The second invention of the present invention is a method for producing a recycled aromatic polyester, including subjecting a polyester containing an aromatic polyester to depolymerization, and then subjecting the component obtained by depolymerization to polycondensation, obtaining a recycled aromatic polyester, in which the polyester containing an aromatic polyester is subjected to depolymerization in an alkylene glycol to obtain an aromatic bis(hydroxyalkyl) dicarboxylate, and the aromatic bis(hydroxyalkyl) dicarboxylate is purified, and the purified aromatic bis(hydroxyalkyl) dicarboxylate is subjected to polycondensation in the presence of an alkali metal and/or an alkaline earth metal, obtaining a recycled aromatic polyester.

The second invention and a preferred embodiment thereof are described in detail below.

### [Polyester]

In the second invention of the present invention, with respect to the polyester as a raw material for obtaining a recycled aromatic polyester, a recovered polyester which contains an aromatic polyester, and which is a polyester recovered from a formed article of a polyester (hereinafter, referred to as "recovered polyester") is used. Examples of formed articles of a polyester include articles of a polyester formed into a fiber, film, sheet, or another form.

When the recovered polyester is a fiber, the fiber may be in a form of a fiber processed article. When the recovered polyester is a film or a sheet, the film or sheet may be in the form of a tray or a bag. As examples of other forms of the recovered polyester, there can be mentioned a form of a bottle.

The recovered polyester may be a polyester recovered from a discarded material or non-confirming article generated in the process for producing the polyester. As examples of such discarded materials and non-confirming articles, there can be mentioned raw material pellets that do not satisfy the required level, unnecessary materials and cut fragments generated during molding or processing, and generated waste, switched products, products made on trial, and defective products generated when changing the grade of products, and cut materials thereof.

In a preferred embodiment of the second invention of the present invention, a waste polyester fiber is used as the recovered polyester. In the recovered polyester, the waste polyester fiber constitutes preferably 50% by mass or more, further preferably 51% by mass or more, further preferably 70% by mass or more of the recovered polyester.

The waste polyester fiber corresponds to the one recovered, before coming into the market as the fiber product or fiber processed article, as a discarded material or the like in the production process, processing step, circulation, or the like, and the fiber product or fiber processed article recovered after being put on the market. The fiber processed article may have been used, or may not have been used. The fiber processed article may be one which is disposed of.

The waste polyester fiber may have a polyester fiber as a constituent fiber, and may further contain a natural fiber, such as cotton, wool, or silk, a chemical fiber derived from a natural material, such as rayon, cupro (cuprammonium rayon), acetate, or lyocell, or a synthetic fiber other than the polyester fiber, such as a polyamide fiber, an acrylic fiber, or an urethane fiber.

The waste polyester fiber may be colored, and may be a cationically dyeable polyester fiber which can be dyed with a cationic dye having excellent color development and clarity. The cationically dyeable polyester fiber is formed from an aromatic polyester having copolymerized an aromatic dicarboxylic acid which has a sulfonate group as a substituent on an aromatic ring, and to which a cationic dye is capable of ionic-bonding.

### [Aromatic polyester]

The aromatic polyester is a polymer obtained by subjecting an aromatic dicarboxylic acid component and a diol component to polycondensation. The aromatic polyester may be either a homopolymer or a copolymer, and may be a mixture thereof.

Examples of the aromatic dicarboxylic acid component constituting the aromatic polyester include terephthalic acid, isophthalic acid, phthalic acid, 2,6-naphthalenedicarboxylic acid, 1,5-naphthalenedicarboxylic acid, bis(p-carboxyphenyl)methane, anthracenedicarboxylic acid, and 4,4'-diphenyl ether dicarboxylic acid, and preferred is terephthalic acid or 2,6-naphthalenedicarboxylic acid, and especially preferred is terephthalic acid.

In addition to the above-mentioned aromatic dicarboxylic acid component, an aromatic dicarboxylic acid having a sulfonate group as a substituent on an aromatic ring, such as sodium 5-sulfoisophthalate, tetrabutylphosphonium 5-sulfoisophthalate, or tetrabutylammonium 5-sulfoisophthalate, may be copolymerized with the aromatic polyester.

With respect to the diol component constituting the aromatic polyester, an aliphatic glycol having 2 to 20 carbon atoms or an alicyclic diol can be used. Specific examples include ethylene glycol, 1,3-propanediol, propylene glycol, 1,4-butanediol, neopentyl glycol, 1,5-pentanediol, 1,6-hexanediol, decamethylene glycol, cyclohexanedimethanol, cyclohexanediol, and dimer diol, and preferred examples include ethylene glycol, 1,3-propanediol, 1,4-butanediol, and 1,4-cyclohexanedimethanol.

A preferred aromatic polyester is a polyalkylene terephthalate using terephthalic acid as a dicarboxylic acid component and using an alkylene glycol as a diol. In the polyalkylene terephthalate, preferred examples of alkylene glycols include ethylene glycol, 1,3-propanediol, and 1,4-butanediol. Preferred examples of the aromatic polyesters include polyethylene terephthalate, polytrimethylene terephthalate, and polybutylene terephthalate. These aromatic polyesters may be a copolymer.

The copolymerized aromatic polyester containing an aromatic dicarboxylic acid having a sulfonate group has excellent dyeability with a cationic dye, and, for this reason, this component is frequently contained in a fiber of aromatic polyester.

In the second invention of the present invention, the aromatic polyester may be a copolymerized aromatic polyester containing, as the dicarboxylic acid component, an isophthalic acid component having a sulfonate group, particularly a metal sulfonate group.

As an example of the isophthalic acid having a metal sulfonate group, there can be mentioned sodium 5-sulfoisophthalate. In this case, the aromatic bis(hydroxyalkyl) dicarboxylate obtained in the below-described depolymerization is sodium bis(2-hydroxyethyl) 5-sulfoisophthalate.

The amount of the sulfoisophthalate component copolymerized in the aromatic polyester is 0.5 to 15 mol%, preferably 1 to 10 mol%, based on the mole of the whole dicarboxylic acid component.

By the second invention of the present invention, a recycled aromatic polyester having high heat resistance can be obtained even when the copolymerized aromatic polyester having the sulfoisophthalate component copolymerized is mixed into the aromatic polyester as a raw material for depolymerization.

Preferred examples of the aromatic polyester as a subject for the second invention of the present invention include an aromatic polyester having terephthalic acid as a main dicarboxylic acid component, sodium 5-sulfoisophthalate as a secondary dicarboxylic acid component, and ethylene glycol as a diol component. The expression "main" indicates 80 mol% or more, preferably 90 mol% or more of the whole dicarboxylic acid component.

### [Depolymerization]

In the second invention of the present invention, the aromatic polyester is subjected to depolymerization in an alkylene glycol. The depolymerization reaction is conducted in the presence of a catalyst, and, as the catalyst, preferably a manganese catalyst or a zinc catalyst, especially preferably a manganese catalyst is used.

Examples of manganese catalysts include a fatty acid salt, a carbonate, a sulfate, a phosphate, an oxide (manganese oxide), a hydroxide, a halide, or an alcoholate of manganese. Examples of fatty acid manganese salts include manganese acetate.

With respect to the manganese catalyst, from the viewpoint of the polymer hue after the repolymerization, manganese oxide or manganese acetate is preferably used, and manganese acetate is especially preferably used. These manganese catalysts may be used individually or in combination. The catalyst is used in the form of a solution or suspension obtained by preliminarily dissolving or suspending the catalyst in an alkylene glycol.

When manganese acetate is used as a catalyst, the catalyst has such high solubility in an alkylene glycol that the amount of the remaining catalyst can be reduced. The amount of the alkylene glycol used in the depolymerization reaction is preferably 2 to 20 times, further preferably 3 to 10 times the mass of the aromatic polyester. When the amount of the alkylene glycol used is in the above range, the amount of the catalyst and other foreign matter mixed into the product can be advantageously reduced.

The amount of the manganese catalyst used in the depolymerization reaction is preferably 20 to 500 mmol%, further preferably 30 to 300 mmol%, especially preferably 50 to 150 mmol%, based on the mole of the whole dicarboxylic acid component constituting the aromatic polyester. When a catalyst other than the manganese catalyst is used, satisfactory depolymerization cannot proceed using the catalyst added in an amount corresponding to such a low concentration. When the amount of the catalyst used is less than 20 mmol%, the catalytic activity is disadvantageously unsatisfactory, and, when the amount of the catalyst used is more than 500 mmol%, a disadvantage is caused in that the effect for suppressing discoloration is small.

The temperature for depolymerization reaction is, for example, 180 to 250°C, preferably 185 to 210°C, and the time for reaction is, for example, 0.5 to 10 hours, preferably 1 to 4 hours. The pressure for reaction may be atmospheric pressure, and may be, for example, 760 to 2,000 mmHg.

### [Alkylene glycol]

With respect to the alkylene glycol, it is preferred that the same alkylene glycol as the diol component constituting the above-mentioned aromatic polyester is used, or the same alkylene glycol as the diol component constituting the recycled aromatic polyester obtained by the method of the second invention of the present invention is used.

That is, it is preferred that, according to the aromatic polyester or recycled aromatic polyester, as the alkylene glycol, those mentioned below are used.

When the aromatic polyester and/or recycled aromatic polyester is polyethylene terephthalate, ethylene glycol is preferably used as the alkylene glycol.

When the aromatic polyester and/or recycled aromatic polyester is polytrimethylene terephthalate, 1,3-propanediol (trimethylene glycol) is preferably used as the alkylene glycol.

When the aromatic polyester and/or recycled aromatic polyester is polybutylene terephthalate, 1,4-butanediol is preferably used as the alkylene glycol.

In any case, the alkylene glycol may be a mixture of alkylene glycols constituting the aromatic polyester and/or recycled aromatic polyester.

### [Purification]

In the second invention of the present invention, it is important that the aromatic bis(hydroxyalkyl) dicarboxylate obtained by subjecting the aromatic polyester to depolymerization is purified.

It is preferred that the purification of the aromatic bis(hydroxyalkyl) dicarboxylate is conducted by crystal deposition in an alkylene glycol solution of the aromatic bis(hydroxyalkyl) dicarboxylate.

The crystal deposition is preferably conducted by decreasing the temperature of the solution, which is obtained by subjecting the aromatic polyester to depolymerization reaction, from a temperature of 60°C or higher as a starting point to a temperature of 25°C or lower, further preferably to a temperature of 15°C or lower.

Decreasing the temperature causes the solid of deposited aromatic bis(hydroxyalkyl) dicarboxylate to be separated from the solution which is a liquid. The solid of deposited aromatic bis(hydroxyalkyl) dicarboxylate is referred to as "cake". The cake is separated from the liquid and then subjected to polycondensation reaction.

An embodiment of the second invention of the present invention is such that the aromatic polyester is an aromatic polyester containing an isophthalic acid component having a metal sulfonate group as a copolymerizable component, that is, the aromatic polyester is an aromatic polyester having terephthalic acid as a main dicarboxylic acid component, sodium 5-sulfoisophthalate as a secondary dicarboxylic acid component, and ethylene glycol as a diol component. In this case, by subjecting the aromatic polyester to depolymerization, a mixture containing a main and secondary depolymerization products is obtained in which the main depolymerization product is bis(2-hydroxyethyl) terephthalate and the secondary depolymerization product is sodium bis(2-hydroxyethyl) 5-sulfoisophthalate. When subjecting the obtained mixture to purification, bis (2-hydroxyethyl) terephthalate (which is frequently referred to as "bis-2-hydroxyethyl terephthalate" or "BHET") is deposited as a cake, and meanwhile, sodium bis(2-hydroxyethyl) 5-sulfoisophthalate (frequently referred to as "BHESI") which is the secondary depolymerization product is not deposited but remains in a liquid state, and therefore, by solid-liquid separation, the deposited BHET can be obtained in a solid state, and separated from BHESI in a liquid state, which is not deposited, so that only BHET with high purity can be removed.

Accordingly, in the second invention of the present invention, even when an aromatic polyester containing an isophthalic acid component having a metal sulfonate group as a copolymerizable component is contained in the aromatic polyester, the aromatic polyester is subjected to depolymerization to separate the copolymerizable component, and subjected to repolycondensation, producing a recycled aromatic polyester having excellent heat resistance. In this instance, the amount of the isophthalic acid component having a metal sulfonate group contained in the aromatic polyester is, for example, 2 mol% or less, preferably 0.5 mol% or less, based on the mole of the whole dicarboxylic acid component of the aromatic polyester.

### [Washing]

The cake of the aromatic bis(hydroxyalkyl) dicarboxylate is preferably washed. That is, it is preferred that, after the crystal deposition, the resultant aromatic bis(hydroxyalkyl) dicarboxylate is washed.

Washing the cake is preferably conducted using water or an alkylene glycol as a washing liquid. The washing liquid preferably has a low viscosity, and, from this point of view, preferred is water. It is preferred that a washing treatment is conducted while spraying the washing liquid onto the cake using a Nutsche (Buchner funnel).

By washing, the depolymerization catalyst dissolved in the alkylene glycol solution after the depolymerization and the discoloration causative agent contained in the aromatic polyester subjected to depolymerization are washed away, making it possible to obtain the aromatic bis(hydroxyalkyl) dicarboxylate having a high purity.

The amount of the washing liquid used in the washing is preferably 1 to 100 times, further preferably 1.5 to 10 times the mass of the cake of the aromatic bis(hydroxyalkyl) dicarboxylate. The temperature of the washing liquid is preferably 0 to 40°C. When the temperature of the washing liquid is higher than 40°C, the cake per se is likely to be dissolved, so that the yield is disadvantageously lowered.

After washing, the cake is dried, so that a high-purity aromatic bis(hydroxyalkyl) dicarboxylate having no sulfo group can be obtained. Drying can be conducted using, for example, a vacuum dryer.

When an alkylene glycol is used as the washing liquid and the alkylene glycol used is the same as the diol component of the recycled aromatic polyester, the aromatic bis(hydroxyalkyl) dicarboxylate may be subjected to polycondensation without being dried.

A preferred embodiment is such that, after being washed, the aromatic bis(hydroxyalkyl) dicarboxylate is dried and then subjected to polycondensation reaction.

### [Polycondensation]

For obtaining a recycled aromatic polyester from the aromatic bis(hydroxyalkyl) dicarboxylate, polycondensation is performed. The polycondensation reaction is conducted using a polymerization catalyst in the presence of an alkali metal and/or an alkaline earth metal. The polycondensation reaction can be conducted using a polymerization catalyst generally used in the art in accordance with the method generally used in the art. It is preferred that the polycondensation reaction is conducted while distilling off an alkylene glycol generated by the polycondensation reaction out of the reaction vessel.

Examples of polymerization catalysts include an antimony catalyst, a germanium catalyst, and a titanium catalyst, and an antimony catalyst is preferably used. As the antimony catalyst, for example, diantimony trioxide is used. The amount of the polymerization catalyst used is preferably 10 to 1,000 ppm, based on the mass of the aromatic bis(hydroxyalkyl) dicarboxylate.

When being subjected to polycondensation reaction, the alkylene glycol content of the purified aromatic bis(hydroxyalkyl) dicarboxylate cake is preferably 100% by mass or less, further preferably 55% by mass or less, further preferably 30% by mass or less, especially preferably 25% by mass or less, based on the mass of the aromatic bis(hydroxyalkyl) dicarboxylate.

The temperature for polycondensation reaction is, for example, 230 to 330°C, preferably 250 to 310°C, and the reaction time is, for example, 240 minutes or less, preferably 180 minutes or less. The pressure for reaction is a reduced pressure, for example, 100 Pa or less, preferably 50 Pa or less.

### [Alkali metal and/or alkaline earth metal]

Examples of alkali metals include lithium, sodium, potassium, rubidium, and cesium, and preferably, sodium or potassium is used.

Examples of alkaline earth metals include calcium, barium, beryllium, and magnesium, and preferably, calcium or magnesium is used.

With respect to the alkali metal and/or alkaline earth metal, especially preferred is sodium.

From the viewpoint of the polymer hue after the repolymerization, the amount of the alkali metal and/or alkaline earth metal used is preferably 5 to 1,000 ppm, further preferably 10 to 500 ppm, based on the mass of the aromatic bis(hydroxyalkyl) dicarboxylate.

The alkali metal and/or alkaline earth metal is preferably used in the form of a salt. That is, the alkali metal and/or alkaline earth metal is preferably used in the form of an alkali metal salt and/or an alkaline earth metal salt.

Examples of acid components constituting the alkali metal salt and/or alkaline earth metal salt include propionic acid, acetic acid, oxalic acid, formic acid, and carbonic acid, and acetic acid is preferably used.

With respect to the alkali metal salt and/or alkaline earth metal salt, preferred is lithium acetate, sodium acetate, potassium acetate, calcium acetate, barium acetate, or magnesium acetate, and sodium acetate is further preferably used.

### [Stabilizer]

In the polycondensation reaction, a phosphorus stabilizer is preferably added. With respect to the phosphorus stabilizer, a known phosphorus stabilizer can be used, and the amount of the phosphorus stabilizer used is, for example, 1 to 100 ppm, based on the mass of the aromatic bis(hydroxyalkyl) dicarboxylate. Examples of phosphorus stabilizers include phosphorous acid, orthophosphoric acid, trimethyl phosphate, triethyl phosphate, triphenyl phosphate, diethyl benzenephosphonate, dimethyl (2-hydroxyethyl)phosphonate and/or phenylphosphonic acid, and preferred examples include orthophosphoric acid, trimethyl phosphate, triethyl phosphate, and triphenyl phosphate. Examples

Hereinbelow, the present invention will be described in more detail with reference to the following Examples. In the following Examples, values were individually determined by the methods for measurement described below.

With respect to the indication for the amount of the catalyst added shown below, "mol%" for the catalyst, based on the mole of the polyester, indicates the ratio of the number of the catalyst molecules to the number of the constituent units of the polyester.

### (1) Methods for measurement

### 1) Hue (L*a*b*)

The measurement was made by the following method. A polymer (5 g) was pressed by two metal plates into a plate form, and then heated at 140°C for 2 hours, crystallizing the sample. With respect to the obtained sample for measurement, using a measurement apparatus ("ZE-6000", manufactured by Nippon Denshoku Industries Co., Ltd.), hue L*, a*, and b* values were measured in accordance with JIS Z8781-4:2013.

### 2) Metal content

The contents of metal elements in a polymer were measured as follows. The polymer was heat-melted to prepare a circular disc, and a content of the metal corresponding to the catalyst used in the disc was individually measured using a fluorescent X-ray measurement apparatus "ZSXPrimus II", manufactured by Rigaku Corporation. In the table below, "ND" means the limit of detection or less (5 ppm or less).

### 3) Intrinsic viscosity (IV)

0.6 g of a polyester was dissolved in 50 cc of o-chlorophenol while heating, and then cooled, and a solution viscosity of the resultant solution was measured using an Ubbelohde viscometer under temperature conditions at 35°C, and, from the measured solution viscosity, an intrinsic viscosity was determined using a calibration curve separately prepared.

### 4) Glass transition point (Tg), melting point (Tm), and crystallization temperature (Tc)

10 mg of a sample was cut and an aluminum pan was filled with the cut sample, and a melting point was measured by means of "DSC apparatus Q10", manufactured by TA Instruments-Waters LLC. Conditions for the measurement were as follows. The temperature of the sample was first increased from 25 to 300°C at a temperature increase rate of 20°C/minute, followed by quenching. Then, the temperature of the quenched sample was increased from 25 to 300°C at 20°C/minute, determining a crystal melting point.

### 5) Sodium 5-sulfoisophthalate content

A sample was formed into a plate, and a sulfur content derived from a sulfo group was measured by means of a fluorescent X-ray apparatus "ZSXPrimus II", manufactured by Rigaku Corporation, and a sodium 5-sulfoisophthalate content was determined. In the table below, "ND" means the limit of detection or less (5 ppm or less).

### 6) Diethylene glycol (DEG) content

Polyester was decomposed using hydrazine hydrate, and a diethylene glycol content of the resultant decomposition product was measured using gas chromatography ("HP6850", manufactured by Hewllet-Packerd Company).

### (2) Polyester resin

As a polyester resin to be subjected to depolymerization, polyethylene terephthalate (PET) pellets, which are generally commercially available, were obtained. The physical properties and color tone of the polyester resin were as shown in Table 1.

In Examples 1 to 3 and Comparative Examples 1 to 12, the polyethylene terephthalate (PET) pellets were used as a polyester resin to be subjected to depolymerization.

**[Table 1]**

| | Physical properties of polyester | | | | Color tone of polyester | | |
|---|---|---|---|---|---|---|---|
| | IV (dl/g) | Tg (°C) | Tc (°C) | Tm (°C) | L* | a* | b* |
| Commercially available PET | 0.64 | 79 | 147 | 256 | 71.7 | -2.5 | -4.2 |

### [Example 1]

300 parts by mass of the polyester resin, 1,500 parts by mass of ethylene glycol (EG), and, as a depolymerization catalyst, 0.38 parts by mass of manganese acetate (100 mmol%, based on the mole of the polyester) were charged into a 2 L separable flask and the flask was purged with nitrogen gas. In this instance, the manganese acetate was preliminarily dissolved in EG and then charged. The polyester resin used was a polyethylene terephthalate resin which is not colored, and contained Sb in an amount of 297 ppm as a polymerization catalyst.

Then, the separable flask containing therein the sample was heated by means of a mantle heater, in which the inner temperature was set to 220°C, and, while stirring, a depolymerization treatment was conducted under atmospheric pressure for 4 hours. The BHET (bis(hydroxyethyl) benzenedicarboxylate) solution obtained after the depolymerization was colorless and transparent, and discoloration was not seen. Further, the solution obtained after the depolymerization was subjected to filtration using a 200 um mesh to remove solids remaining in the solution, and the resultant solution was gradually cooled to 70°C and then, while stirring and cooling, subjected to temperature decrease of from 70°C to 40°C from a time of 0 to 10 minutes, temperature decrease of from 40°C to 30°C from a time of 10 to 60 minutes, and temperature decrease of from 30°C to 15°C from a time of 60 to 180 minutes, and then the resultant solution was stirred for 60 minutes while maintaining the inner temperature at 15°C, and the inner temperature was decreased to cause crystals of BHET to deposit (for 4 hours in total), obtaining a BHET/EG slurry.

The BHET/EG slurry was subjected to press treatment using a filter press, manufactured by Nihon Rokasochi Co., Ltd., to perform solid-liquid separation of BHET and EG. The BHET separated in this instance contained EG in an amount of 35% by mass, based on the weight of the cake recovered after the filter press treatment. The cake obtained after the EG separation was subjected to water washing treatment using a Nutsche while spraying pure water at 25°C in an amount two times the weight of the cake. The BHET obtained after completion of the solid-liquid separation was then subjected to drying treatment in a vacuum dryer at 50°C for 8 hours, obtaining dried BHET. The obtained BHET was white, and mixing of foreign matter was not seen.

Then, 254 parts by mass of the obtained dried BHET and 0.007 parts by mass of a phosphorus stabilizer as well as 0.07 parts by mass of diantimony trioxide as a repolymerization catalyst were charged into a reaction vessel in a nitrogen gas atmosphere under atmospheric pressure. Then, the temperature in the reaction vessel was set to 285°C and the pressure was stepwise reduced under conditions at atmospheric pressure for 10 minutes, a pressure of 4 kPa for 10 minutes, and a pressure of 0.4 kPa for 40 minutes, and, while distilling off ethylene glycol and the like generated by the reaction out of the reaction vessel, a polycondensation reaction was conducted.

The quality of the finally formed polyethylene terephthalate was shown in Tables 2 and 3.

**[Table 2]**

| | Depolymerizati on catalyst | BHET Solution | Residual catalyst metal content (ppm) | |
|---|---|---|---|---|
| | | | Depolymerization catalyst | Polymerization catalyst |
| Example 1 | Manganese acetate | Colorless | ND | 260 |
| Example 2 | Manganese oxide | Colorless | 9 | 346 |
| Example 3 | Manganese acetate | Colorless | ND | 271 |
| Comparative Example 1 | Calcium acetate | Discolored | 22 | 390 |
| Comparative Example 2 | Magnesium acetate | Discolored | 29 | 351 |
| Comparative Example 3 | Cerium acetate | Discolored | 226 | 369 |
| Comparative Example 4 | Cobalt acetate | Discolored | 141 | 364 |
| Comparative Example 5 | Titanium tetrabutoxide | Discolored | 193 | 103 |
| Comparative Example 7 | Sodium hydroxide | Discolored | 199 | 352 |
| Comparative Example 8 | Sodium carbonate | Discolored | 139 | 276 |
| Comparative Example 9 | Sodium acetate | Discolored | 47 | 355 |
| Comparative Example 11 | Potassium carbonate | Discolored | 84 | 270 |
| Comparative Example 12 | Potassium acetate | Discolored | 98 | 394 |

**[Table 3]**

| | Physical properties of repolymerized polyester | | | | Color tone of repolymerized polyester | | |
|---|---|---|---|---|---|---|---|
| | IV (dl/g) | Tg (°C) | Tc (°C) | Tm (°C) | L* | a* | b* |
| Example 1 | 0.64 | 79 | 146 | 254 | 70.0 | -2.5 | -4.0 |
| Example 2 | 0.62 | 79 | 138 | 253 | 74.1 | -2.3 | -3.7 |
| Example 3 | 0.63 | 78 | 144 | 253 | 69.4 | -2.7 | -4.1 |
| Comparative Example 1 | 0.63 | 78 | 147 | 256 | 76.5 | -2.7 | -2.8 |
| Comparative Example 2 | 0.65 | 79 | 152 | 255 | 75.8 | -2.5 | -2.7 |
| Comparative Example 3 | 0.64 | 79 | 150 | 256 | 75.6 | -2.8 | -2.5 |
| Comparative Example 4 | 0.62 | 78 | 146 | 254 | 65.6 | -1.0 | -1.1 |
| Comparative Example 5 | 0.64 | 79 | 146 | 254 | 66.9 | -0.4 | 16.7 |
| Comparative Example 7 | 0.63 | 78 | 141 | 257 | 73.3 | -3.3 | -0.8 |
| Comparative Example 8 | 0.64 | 79 | 143 | 256 | 72.0 | -3.2 | -0.9 |
| Comparative Example 9 | 0.64 | 79 | 148 | 256 | 74.7 | -3.1 | 1.7 |
| Comparative Example 11 | 0.66 | 79 | 145 | 255 | 65.5 | -2.2 | 1.3 |
| Comparative Example 12 | 0.63 | 79 | 148 | 254 | 66.2 | -2.7 | 2.1 |

### [Example 2]

Substantially the same treatment as in Example 1 was performed except that, instead of the manganese acetate used as a depolymerization catalyst in Example 1, manganese oxide was used, and the BHET obtained by depolymerization and the polyethylene terephthalate resin obtained by repolymerization were sampled. In this instance, the BHET solution obtained after the depolymerization was colorless and transparent, and discoloration was not seen. The quality was shown in Tables 2 and 3.

### [Example 3]

Substantially the same treatment as in Example 1 was performed except that, with respect to the manganese acetate used in Example 1, the amount of the manganese acetate added was changed from 0.38 parts by mass (100 mmol%, based on the mole of the polyester) to 0.30 parts by mass (80 mmol%, based on the mole of the polyester). In the BHET solution obtained after the depolymerization, a slight amount of an undissolved material was found, but the BHET obtained by depolymerization and the polyethylene terephthalate resin obtained by repolymerization were able to be sampled. The quality was shown in Tables 2 and 3.

Substantially the same treatment as in Example 1 was performed except that, with respect to the manganese acetate used in Example 1, the amount of the manganese acetate added was further reduced to 0.038 parts by mass (10 mmol%, based on the mole of the polyester), but depolymerization did not satisfactorily proceed, so that a large amount of the undecomposed polyester remained in the flask. The amount of the obtained BHET was too small to conduct repolymerization.

### [Comparative Examples 1 to 4]

Substantially the same treatment as in Example 1 was performed except that, instead of the manganese acetate used as a depolymerization catalyst in Example 1, calcium acetate (Comparative Example 1), magnesium acetate (Comparative Example 2), cerium acetate (Comparative Example 3), or cobalt acetate (Comparative Example 4) was used, and the BHET obtained by depolymerization and the polyethylene terephthalate resin obtained by repolymerization were sampled. When using calcium acetate (Comparative Example 1) or magnesium acetate (Comparative Example 2), a relatively large amount of undecomposed materials were seen after the depolymerization. Further, with respect to the BHET solution obtained after the depolymerization in each of Comparative Examples 1 to 4, unlike the Examples, discoloration was seen. The physical properties of the finally obtained polyester, such as a melting point, were equivalent to those of the Examples, but discoloration was observed like the BHET in an intermediate stage. Further, the residual metal contents derived from the catalyst components were high, as compared to that for manganese. The quality was shown in Tables 2 and 3.

### [Comparative Example 5]

Substantially the same treatment as in Example 1 was performed except that, instead of the manganese acetate used in Example 1, titanium tetrabutoxide was used as a depolymerization catalyst, and that this catalyst is also a polymerization catalyst and hence 0.07 g of diantimony trioxide which is a polymerization catalyst was not added, and the BHET obtained by depolymerization and the polyethylene terephthalate resin obtained by repolymerization were sampled. With respect to the BHET solution obtained after the depolymerization, severe discoloration was seen. The antimony content of the finally obtained polyester resin was suppressed, but the residual metal content for titanium was high, as compared to that for manganese, and it was impossible to suppress discoloration of the BHET and resin. The quality was shown in Tables 2 and 3.

### [Comparative Example 6]

Substantially the same treatment as in Example 1 was performed except that, instead of the manganese acetate used in Example 1, sodium hydroxide was used as a depolymerization catalyst. However, depolymerization did not satisfactorily proceed, so that a large amount of the undecomposed polyester remained in the flask. The amount of the BHET was too small to conduct repolymerization.

### [Comparative Examples 7 to 9]

Substantially the same treatment as in Example 1 was performed except that, instead of the manganese acetate used in Example 1, sodium hydroxide like Comparative Example 6 (Comparative Example 7), sodium carbonate (Comparative Example 8), or sodium acetate (Comparative Example 9) was used as a depolymerization catalyst, and that the amount of the catalyst charged was changed from 0.38 parts by mass in Example 1 to 9.0 parts by mass, and the BHET obtained by depolymerization and the polyethylene terephthalate resin obtained by repolymerization were sampled. With respect to the BHET solution obtained after the depolymerization, discoloration was seen. However, as a result, the residual metal content of the repolymerized polyester was larger than that in the Examples, and discoloration was also seen in the polyester. The quality was shown in Tables 2 and 3.

### [Comparative Example 10]

Substantially the same treatment as in Example 1 was performed except that, instead of the manganese acetate used in Example 1, potassium carbonate was used as a depolymerization catalyst. However, depolymerization did not satisfactorily proceed, so that a large amount of the undecomposed polyester remained in the flask. The amount of the BHET was too small to conduct repolymerization.

### [Comparative Examples 11 and 12]

Substantially the same treatment as in Example 1 was performed except that, instead of the manganese acetate used in Example 1, potassium carbonate like Comparative Example 10 (Comparative Example 11), or potassium acetate (Comparative Example 12) was used as a depolymerization catalyst, and that the amount of the catalyst charged was changed from 0.38 parts by mass in Example 1 to 9.0 parts by mass, and the BHET obtained by depolymerization and the polyethylene terephthalate resin obtained by repolymerization were sampled. With respect to the BHET solution obtained after the depolymerization, discoloration was seen. However, as a result, the residual metal content of the repolymerized polyester was larger than that of the Examples, and discoloration was also seen in the polyester. The quality was shown in Tables 2 and Table 3.

### [Reference Example]

Hereinbelow, productions of the copolymerized aromatic polyester and aromatic polyester used in Example 21, Example 22, Reference Example 21, and Comparative Example 21 are described in Reference Examples 1 to 3.

### [Reference Example 1] (Production 1 of sodium 5-sulfoisophthalate copolymerized aromatic polyester)

191.3 parts by mass of dimethyl terephthalate (DMT), 124.2 parts by mass of ethylene glycol (EG), and 4.4 parts by mass of dimethyl sodium 5-sulfoisophthalate, manufactured by Takemoto Oil & Fat Co., Ltd., were placed in a reaction vessel in a nitrogen gas atmosphere under atmospheric pressure, and 0.07 parts by mass of manganese acetate and 0.22 parts by mass of sodium acetate were added as a catalyst, and the resultant mixture was subjected to transesterification reaction at a reaction temperature of 240°C while removing methanol.

Then, into the reaction product obtained after completion of the transesterification reaction were charged 0.07 parts by mass of diantimony trioxide and 0.03 parts by mass of orthophosphoric acid. The temperature in the reaction vessel was set to 285°C and the pressure was stepwise reduced under conditions at atmospheric pressure for 10 minutes, a pressure of 4 kPa for 10 minutes, and a pressure of 0.4 kPa for 40 minutes, and, while distilling off ethylene glycol and the like generated by the reaction out of the reaction vessel, a polycondensation reaction was conducted. Clogging the distillate outlet with an oligomer or the like did not occur. The quality of the formed copolymerized aromatic polyester is shown in Table 4.

### [Reference Example 2] (Production 2 of sodium 5-sulfoisophthalate copolymerized aromatic polyester)

A transesterification reaction was conducted under substantially the same conditions as those in Reference Example 1 except that the amount of dimethyl terephthalate (DMT) was changed to 185.7 parts by mass, and that the amount of dimethyl sodium 5-sulfoisophthalate was changed to 13.0 parts by mass, obtaining a copolymerized aromatic polyester. The quality of the obtained aromatic polyester was shown in Table 4.

### [Reference Example 3] (Production of an aromatic polyester)

194.2 parts by mass of dimethyl terephthalate (DMT), 124.2 parts by mass of ethylene glycol (EG), and 0.07 parts by mass of manganese acetate were placed in a reaction vessel in a nitrogen gas atmosphere under atmospheric pressure, and the resultant mixture was subjected to transesterification reaction at a reaction temperature of 240°C while distilling off methanol.

Then, into the reaction product obtained after completion of the transesterification reaction were charged 0.07 parts by mass of diantimony trioxide and 0.03 parts by mass of orthophosphoric acid. The temperature in the reaction vessel was set to 285°C and the pressure was stepwise reduced under conditions at atmospheric pressure for 10 minutes, a pressure of 4 kPa for 10 minutes, and a pressure of 0.4 kPa for 40 minutes, and, while distilling off ethylene glycol and the like generated by the reaction out of the reaction vessel, a polycondensation reaction was conducted. Clogging the distillate outlet with an oligomer or the like did not occur. The quality of the formed aromatic polyester was shown in Table 4.

### [Example 21]

300 parts by mass of the copolymerized aromatic polyester obtained in Reference Example 1, 1,500 parts by mass of ethylene glycol (EG), and 0.38 parts by mass of manganese acetate (100 mmol%, based on the mole of the whole dicarboxylic acid component of the copolymerized aromatic polyester) were charged into a 2 L separable flask.

In this instance, the manganese acetate was preliminarily dissolved in EG and used in the form of a solution. Then, the flask was purged with nitrogen gas and, while stirring, a depolymerization reaction was conducted at 202°C for 4 hours, obtaining a depolymerization sample.

The obtained depolymerization sample was gradually cooled to 70°C and then, while stirring and cooling using a thermostatic chamber at 15°C, the inner temperature was decreased to cause crystals of bis-2-hydroxyethyl terephthalate (BHET) to deposit, obtaining a BHET/EG slurry.

The BHET/EG slurry was subjected to press treatment using a filter press, manufactured by Nihon Rokasochi Co., Ltd., to perform solid-liquid separation of BHET and EG, obtaining a cake of BHET. The BHET obtained in this instance contained EG in an amount of 34% by mass, based on the mass of the cake recovered after the filter press treatment.

The cake of BHET obtained after the EG separation was subjected to water washing treatment using a Nutsche while spraying pure water in an amount two times the mass of the cake. The cake of BHET obtained after completion of the solid-liquid separation was subjected to drying treatment in a vacuum dryer at 50°C for 8 hours, obtaining a depolymerization sample 1.

Then, 254 parts by mass of the obtained depolymerization sample 1, 0.07 parts by mass of diantimony trioxide, 0.005 parts by mass of orthophosphoric acid, and 0.22 parts by mass of sodium acetate were charged into a reaction vessel in a nitrogen gas atmosphere under atmospheric pressure.

Then, the temperature in the reaction vessel was set to 285°C and the pressure was stepwise reduced under conditions at atmospheric pressure for 10 minutes, a pressure of 4 kPa for 10 minutes, and a pressure of 0.4 kPa for 40 minutes, and, while distilling off ethylene glycol and the like generated by the reaction out of the reaction vessel, a polycondensation reaction was conducted, obtaining a recycled aromatic polyester.

Despite having experienced depolymerization and polycondensation, the obtained recycled aromatic polyester had higher IV and melting point than those of the aromatic polyester as a raw material, and was a white high-quality recycled aromatic polyester. The quality of the recycled aromatic polyester is shown in Table 5.

### [Example 22]

A depolymerization reaction was conducted in substantially the same manner as in Example 21 except that the copolymerized aromatic polyester of Reference Example 1 used in Example 21 was changed to the copolymerized aromatic polyester of Reference Example 2, obtaining a depolymerization sample 2.

254 parts by mass of the obtained depolymerization sample 2, 0.07 parts by mass of diantimony trioxide, 0.005 parts by mass of orthophosphoric acid, and 0.22 parts by mass of sodium acetate were charged into a reaction vessel in a nitrogen gas atmosphere under atmospheric pressure.

Then, the temperature in the reaction vessel was set to 285°C, and the pressure was stepwise reduced under conditions at atmospheric pressure for 10 minutes, a pressure of 4 kPa for 10 minutes, and a pressure of 0.4 kPa for 40 minutes, and, while distilling off ethylene glycol and the like generated by the reaction out of the reaction vessel, a polycondensation reaction was conducted, obtaining a recycled aromatic polyester.

Despite having experienced depolymerization and polycondensation, the obtained recycled aromatic polyester had higher IV and melting point than those of the aromatic polyester as a raw material, and was a white high-quality recycled aromatic polyester. The quality of the recycled aromatic polyester was shown in Table 5.

### [Reference Example 21]

300 parts by mass of the copolymerized aromatic polyester obtained in Reference Example 1, 1,500 parts by mass of ethylene glycol (EG), and 0.38 parts by mass of manganese acetate were charged into a separable flask having a capacity of 2 L, and the flask was purged with nitrogen gas and, while stirring, a depolymerization reaction was conducted at 202°C for 4 hours, obtaining a depolymerization sample.

508 parts by mass of the above-obtained depolymerization sample, 0.07 parts by mass of diantimony trioxide, 0.005 parts by mass of orthophosphoric acid, and 0.22 parts by mass of sodium acetate were charged into a reaction vessel in a nitrogen gas atmosphere under atmospheric pressure.

Then, the temperature in the reaction vessel was set to 285°C, and the pressure was stepwise reduced under conditions at atmospheric pressure for 10 minutes, a pressure of 4 kPa for 10 minutes, and a pressure of 0.4 kPa for 40 minutes, and, while distilling off ethylene glycol and the like generated by the reaction out of the reaction vessel, a polycondensation reaction was conducted, obtaining a recycled aromatic polyester.

The obtained recycled aromatic polyester had a large residual Na sulfoisophthalate content, and had lower IV value and melting point than those in Examples 21 and 22. The quality of the recycled aromatic polyester was shown in Table 5.

### [Comparative Example 21]

254 parts by mass of the depolymerization sample 2 obtained in Example 22, 0.07 parts by mass of diantimony trioxide, and 0.005 parts by mass of orthophosphoric acid were charged into a reaction vessel in a nitrogen gas atmosphere under atmospheric pressure.

Then, the temperature in the reaction vessel was set to 285°C and the pressure was stepwise reduced under conditions at atmospheric pressure for 10 minutes, a pressure of 4 kPa for 10 minutes, and a pressure of 0.4 kPa for 40 minutes, and, while distilling off ethylene glycol and the like generated by the reaction out of the reaction vessel, a polycondensation reaction was conducted, obtaining a recycled aromatic polyester.

The obtained recycled aromatic polyester had a small sodium 5-sulfoisophthalate content, but had a low melting point. The quality of the recycled aromatic polyester was shown in Table 5.

**[Table 4]**

| | IV (dl/g) | Sodium 5-sulfoisophthalate component content (mol%) | Tm (°C) | DEG (mass%) |
|---|---|---|---|---|
| Reference Example 1 | 0.53 | 1.5 | 250 | 1.1 |
| Reference Example 2 | 0.37 | 4.3 | 246 | 2.2 |
| Reference Example 3 | 0.65 | 0 | 256 | 0.8 |

**[Table 5]**

| | IV (dl/g) | Sodium 5-sulfoisophthalate component content (mol%) | Tm (°C) | DEG (mass%) |
|---|---|---|---|---|
| Example 21 | 0.65 | ND | 255 | 0.9 |
| Example 22 | 0.65 | 0.2 | 254 | 0.9 |
| Reference Example 21 | 0.55 | 1.4 | 250 | 1.2 |
| Comparative Example 21 | 0.69 | 0.2 | 219 | 9.0 |

### Industrial Applicability

The aromatic bis(hydroxyalkyl) dicarboxylate obtained by the method of the present invention, an aromatic polyester obtained from the aromatic bis(hydroxyalkyl) dicarboxylate as at least one raw material, and a recycled aromatic polyester, which is obtained by subjecting an aromatic polyester component contained in waste polyester, a recovered product, or the like to depolymerization and subjecting the resultant component to repolymerization, have small yellowness and excellent hue, and can suppress deterioration of the physical properties by virtue of the presence of a copolymerizable component before depolymerization, and therefore can be advantageously used as a material for, for example, a fiber, a film, and a resin.

## Claims

1. A method for producing an aromatic bis(hydroxyalkyl) dicarboxylate, comprising subjecting a polyester to depolymerization in the presence of a catalyst, wherein the catalyst is a manganese catalyst, and wherein the amount of the catalyst used is 20 to 500 mmol%, based on the mole of the polyester.

2. The method for producing an aromatic bis(hydroxyalkyl) dicarboxylate according to claim 1, wherein the polyester is comprised mainly of a polyalkylene terephthalate.

3. The method for producing an aromatic bis(hydroxyalkyl) dicarboxylate according to claim 2, wherein the polyalkylene terephthalate is one of polyethylene terephthalate, polytrimethylene terephthalate, and polybutylene terephthalate.

4. The method for producing an aromatic bis(hydroxyalkyl) dicarboxylate according to claim 1, wherein the aromatic bis(hydroxyalkyl) dicarboxylate is a bis(hydroxyalkyl) benzenedicarboxylate.

5. The method for producing an aromatic bis(hydroxyalkyl) dicarboxylate according to claim 1, wherein the manganese catalyst is manganese acetate.

6. The method for producing an aromatic bis(hydroxyalkyl) dicarboxylate according to claim 1, wherein the catalyst is used in the form of a solution obtained by preliminarily dissolving the catalyst in an alkylene glycol.

7. The method for producing an aromatic bis(hydroxyalkyl) dicarboxylate according to claim 1, wherein, after the depolymerization, the aromatic bis(hydroxyalkyl) dicarboxylate is subjected to crystal deposition in an alkylene glycol by decreasing the temperature.

8. The method for producing an aromatic bis(hydroxyalkyl) dicarboxylate according to claim 7, wherein, after the crystal deposition, the resultant aromatic bis(hydroxyalkyl) dicarboxylate is washed with water.

9. A method for producing a polyester resin, comprising subjecting to repolymerization the aromatic bis(hydroxyalkyl) dicarboxylate obtained by the method according to any one of claims 1 to 8.

10. A polyester resin which is obtained by the method for producing a polyester resin according to claim 9.

11. A method for producing a recycled aromatic polyester, comprising subjecting a polyester containing an aromatic polyester to depolymerization, and then subjecting the component obtained by depolymerization to polycondensation, obtaining a recycled aromatic polyester,
wherein the depolymerization is performed by conducting depolymerization of the polyester in the presence of a catalyst to obtain an aromatic bis(hydroxyalkyl) dicarboxylate, wherein the catalyst used in the depolymerization is a manganese catalyst, and wherein the amount of the catalyst used is 20 to 500 mmol%, based on the mole of the polyester.

12. The method for producing a recycled aromatic polyester according to claim 11, wherein, after the depolymerization and before the polycondensation, the aromatic bis(hydroxyalkyl) dicarboxylate is purified.

13. The method for producing a recycled aromatic polyester according to claim 12, wherein the polycondensation is performed by conducting polycondensation of the purified aromatic bis(hydroxyalkyl) dicarboxylate in the presence of an alkali metal and/or an alkaline earth metal.

14. A method for producing a recycled aromatic polyester, comprising subjecting a polyester containing an aromatic polyester to depolymerization, and then subjecting the component obtained by depolymerization to polycondensation, obtaining a recycled aromatic polyester, wherein the polyester containing an aromatic polyester is subjected to depolymerization in an alkylene glycol to obtain an aromatic bis(hydroxyalkyl) dicarboxylate, and the aromatic bis(hydroxyalkyl) dicarboxylate is purified, and the purified aromatic bis(hydroxyalkyl) dicarboxylate is subjected to polycondensation in the presence of an alkali metal and/or an alkaline earth metal, obtaining a recycled aromatic polyester.

15. The method according to claim 14, wherein the polyester is a recovered polyester.

16. The method according to claim 14, wherein the purification of the aromatic bis(hydroxyalkyl) dicarboxylate is conducted by crystal deposition in an alkylene glycol solution of the aromatic bis(hydroxyalkyl) dicarboxylate.

17. The method according to claim 16, wherein, after the crystal deposition, the resultant aromatic bis(hydroxyalkyl) dicarboxylate is washed.

18. The method according to claim 17, wherein the washing is performed using water or an alkylene glycol.

19. The method according to claim 16, wherein the crystal deposition is conducted by decreasing the temperature of the solution, which is obtained by subjecting the aromatic polyester to depolymerization reaction, from a temperature of 60°C or higher as a starting point to a temperature of 25°C or lower.

20. The method according to claim 17, wherein, after being washed, the aromatic bis(hydroxyalkyl) dicarboxylate is dried, and then subjected to polycondensation reaction.

21. A recycled aromatic polyester which is obtained by the method according to claim 14.

22. The method according to claim 14, wherein the alkylene glycol content of the purified aromatic bis(hydroxyalkyl) dicarboxylate is 100% by mass or less, and the aromatic bis(hydroxyalkyl) dicarboxylate in this state is subjected to polycondensation.
